# FASCICULE DE BREVET EUROPEEN

(11) **EP 1 685 810 B1**
(45) Date de publication et mention de la délivrance du brevet: **21.10.2009**
(21) Numéro de dépôt: 05100624.5
(22) Date de dépôt: 31.01.2005
(51) Int. Cl.: A61F 2/34

(54) **Conformation particulière d'un élément d'ancrage acétabulaire**
Besondere Gestaltung eines Verankerungselements einer Hüftpfanne
Particular conformation of an acetabular anchoring element

(43) Date de publication de la demande: 02.08.2006
(73) Titulaire: Bone and Joint Research S.A., 3650 Kayl (LU)
(72) Inventeur: Autrique, Jean-Claude Dr., 1300 WAVRE (BE); Oosterlinck, Dirk Dr., 8792 WAREGEM (BE); Poilvache, Pascal Dr., 1060 BRUXELLES (BE); Thiéry, Jacques Dr., 1390 GREZ-DOICEAU (BE); Vander Maren, Christian Dr., 1340 OTTIGNIES (BE); Goube, Michel, 62152 NEUFCHATEL HARDELOT (FR); Bigand, Dominique, 62170 Inxent (FR); Thiltges, Paul, 1020 BRUXELLES (BE); Thiltges, Laurence, 1020 BRUXELLES (BE); Bogaert, Johan Dr., 3540 HERK-DE-STAD (BE); Boucquey, Pierre Dr., 7700 MOUSCRON (BE); Deneufbourg, Jean Dr., 1300 WAVRE (BE); Geulette, Bernard Dr., 1640 RHODE ST GENESE (BE); Ghijselinqs, Ignace Dr., 9910 URSEL (BE); Ghosez, Jean-Pierre Dr., 5081 BOVESSE (BE); Legaye, Jean Dr., 5340 GESVES (BE); Manche, Eric Dr., 7063 Soignies (BE)
(74) Mandataire: Machtalère, Georges

(56) Documents cités:
- EP-A- 0 617 932
- EP-A- 1 086 666
- EP-A- 1 114 624
- FR-A- 2 639 822
- FR-A- 2 749 162

## Description

La présente invention porte sur une conformation particulière d'un élément d'ancrage acétabulaire élastique pour prothèse totale de hanche.

Dans les pathologies de l'articulation de la hanche, il est maintenant d'usage d'utiliser des implants d'ancrage des deux côtés de l'articulation, à savoir des tiges fémorales dans le fémur, et des implants d'ancrage acétabulaire (cupules et/ou inserts) dans la cavité cotyloidienne.

L'interface entre tige fémorale et ancrage acétabulaire est constituée par une bille sphérique fémorale et un insert fixé en général à l'élément d'ancrage acétabulaire.

L'ancrage dans la cavité cotyloïdienne est constitué par un implant métallique, plastique (polyéthylène) ou autres matériaux biocompatibles que l'on appelle souvent la cupule.

Pour que l'élément d'ancrage cotyloïdien soit stable, plusieurs solutions ont été réalisées :
- ancrage à l'aide de vis qui le traversent et le fixent à l'os iliaque
- ancrage à l'aide de ciment
- par picots sur la cupules
- ancrage par fusion osseuse de la surface externe de l'élément d'ancrage acétabulaire avec la cavité cotyloïdienne, la surface externe étant constituée par un recouvrement réalisant cette fusion (par exemple de l'hydroxy-apatite).

Les trois premiers ancrages assurent la stabilité primaire de l'élément d'ancrage acétabulaire (stabilité en post-opératoire immédiat). Le dernier ancrage (par fusion osseuse) est un ancrage qui se réalise quelques mois après; il assure donc un ancrage secondaire.

Ces ancrages sont solides au sens mécanique du terme, c'est-à-dire qu'ils n'admettent pas de déformation équivalente en liaison avec l'os receveur. Il y a donc une hétérogénéité de comportement mécanique entre l'os et l'élément d'ancrage acétabulaire. Les modules respectifs d'élasticité de l'élément d'ancrage acétabulaire et de l'os avoisinant sont très différents. Cela n'est pas une bonne conformation pour la transmission d'efforts dynamiques et de chocs. Il est certain que l'insert en polyéthylène absorbe les chocs mais il a la fâcheuse tendance à fluer.

Des fabricants ont donc installé sur les éléments d'ancrage acétabulaire des systèmes de fentes.

L'avantage à tirer de ces fentes est de proposer une certaine élasticité au niveau du pourtour équatorial de la cavité cotyloïdienne, et en particulier en face des cornes, qui sont des éléments osseux les plus rigides sur ledit pourtour.

Aussi les dispositions de fentes des cupules existantes sont loin de pouvoir entraîner cet avantage.

En effet la fente doit impérativement être équatoriale; il n'y a aucun intérêt en effet à ce qu'elle soit dans la zone polaire de l'élément d'ancrage acétabulaire puisque le fond de la cavité cotyloïdienne est plus faible mécaniquement; d'autre part, pour fonctionner, elle ne doit pas déformer l'intérieur de l'élément d'ancrage acétabulaire sous peine d'apporter des contraintes supplémentaires et parasites à l'insert ; enfin elles ne peuvent fonctionner que si elles sont, non pas en retrait ou au niveau de la surface générale équatoriale, mais en relief ou excentrées par rapport à la surface équatoriale moyenne.

Ce problème a été traité de différente manière:

Afin de diminuer la raideur de la cupule, certains implants métalliques possèdent des systèmes de fentes disposés de différentes façons suivant le fabriquant.

Les unes sont débouchantes au travers de l'élément d'ancrage acétabulaire; le système de fentes utilise la totalité de l'épaisseur (par exemple FR2721201).

Les autres sont constituées de fentes situées dans l'épaisseur même de l'élément d'ancrage acétabulaire (par exemple FR2798583 qui représente l'art antérieur le plus proche).

Pour les premiers systèmes, les fentes sont soit méridiennes, et partent depuis l'équateur pour atteindre la proximité du pôle, d'autres sont disposées à des latitudes moyennes. Dans ce cas, la fente est suivant une longitude de la cupule, et la déformation intéresse toute la cupule.

Enfin pour les seconds systèmes, les fentes sont plutôt équatoriales et dans l'épaisseur.

Pour le premier système, l'inconvénient provient du fait que les fentes mettent en mouvement aussi bien l'extérieur de la cupule que l'intérieur ; ces déformations entraînent des sur-contraintes sur l'insert et parfois son expulsion.

Pour les seconds systèmes, les fentes sont réalisées dans l'épaisseur de la cupule au niveau de l'équateur; aussi la surface externe au regard des parties fendues, donc des parties de la cupule susceptibles de se déformer, est située sur la sphère de la zone équatoriale de l'élément d'ancrage acétabulaire, c'est-à-dire sans être au-delà ni en deçà de la cote représentée par le diamètre de la cupule au niveau équatorial.

Ainsi lorsque l'élément d'ancrage acétabulaire est impacté dans la cavité cotyloïdienne, à savoir une portion de sphère dans une autre portion de sphère légèrement plus petite, alors, compte tenu que les zones en regard des fentes ne sont pas en sur-épaisseur ou excentrées, l'appui se fait partout de la même manière, uniformément, et ainsi les fentes ne se déforment pas; elles ne jouent donc aucun rôle dans la stabilité primaire.

Le brevet FR2733904 décrit une cupule souple en deux parties dont la partie externe est la seule déformable. La déformation intéresse toute la zone externe de la cupule. Elle ne constitue pas une cupule à déformation locale.

Ainsi nous avons soit affaire à des systèmes de fentes qui occupent toute la cupule en traversant celle-ci dans l'épaisseur, soit des systèmes de fentes créées dans l'épaisseur même de la cupule, donc parallèles à la surface externe de la cupule sans jamais la dépasser, ne pouvant donc pas créer de véritable déformation.

L'invention suivante permet de remédier à ces inconvénients.

L'élément d'ancrage acétabulaire comporte classiquement une surface interne qui reçoit des inserts et une surface externe dont l'enveloppe géométrique est constituée de différentes portions de sphères de rayon différent, la dernière portion située à l'équateur, comportant un système de lamelles disposées suivant une ligne en arc de cercle, chaque lamelle étant disposée en sur-épaisseur par rapport à la surface équatoriale avoisinant ces dites lamelles. L'objet de l'invention réside dans le fait que la fente est pratiquée dans la sur-épaisseur et non dans l'épaisseur, générant une lamelle qui se situe en sur-élévation par rapport à la ligne équatoriale de la surface externe de la cupule. Etant en sur-épaisseur, cette lamelle sera le premier élément en contact avec la cavité cotyloïdienne.

La lamelle est le résultat de la pratique d'une fente dans l'épaisseur équatoriale de l'élément d'ancrage acétabulaire. La fente débouche soit latéralement et vers le bas, c'est-à-dire dans la partie inférieure de l'élément d'ancrage acétabulaire, soit unilatéralement et vers le bas.

D'une part, on est donc en présence d'un système de lamelles déformables du fait de la présence de fentes, et d'autre part, la surface externe de chaque lamelle est en sur-épaisseur ou dans une position excentrée par rapport à la zone équatoriale avoisinant les lamelles.

Cette combinaison relief ou sur-épaisseur et fente permet lors de l'impaction de la cupule, que les lamelles soient d'abord en contact les premières sur le pourtour de la cavité cotyloïdienne, puis se déforment jusqu'au moment où prend fin l'impaction.

En particulier, on peut réaliser un système de une ou deux fentes qui seront en regard des cornes de la cavité cotyloïdienne. Ainsi la déformation et l'élasticité sont sur un ou les deux points durs du pourtour cotyloïdien.

L'épaisseur de la fente et la hauteur du relief sont telles que la déformation initiale n'entame qu'une partie du potentiel de déformation.

Ainsi on assure une stabilité primaire à la cupule dans son logement, mais encore une certaine élasticité résiduelle de la cupule au regard de certaines parties de la cavité cotyloïdienne, en particulier des cornes. Enfin l'ancrage de l'insert dans l'élément d'ancrage acétabulaire reste rigide quelles que soient les déformations externes.

Selon une caractéristique de l'invention, l'implant d'ancrage acétabulaire (1) destiné à être implanté dans la cavité cotyloïde, comporter une surface interne (2) qui reçoit des inserts et une surface externe (3) dont l'enveloppe géométrique est constituée de plusieurs portions (4) de surfaces ou de sphères de rayon différent, la dernière portion (5) située à l'équateur comportant une ou plusieurs lamelles (6) disposées suivant un arc de cercle, caractérisé en ce que ces dites lamelles (6) possèdent une surface externe (7) en sur-élévation ou sur-épaisseur par rapport à la surface externe (8) de la portion (5) de l'élément d'ancrage acétabulaire.

Selon une autre caractéristique de l'invention, l'implant d'ancrage acétabulaire (1) selon la caractéristique précédente, est caractérisé en ce que la fente (9) pratiquée sous les lamelles (6) détermine des ouvertures (10) sur les côtés (11) et une ouverture (12) sur le côté (13) de l'élément d'ancrage acétabulaire (1).

Selon une autre caractéristique de l'invention, l'implant d'ancrage acétabulaire (1') destiné à être implanté dans la cavité cotyloïde, comportant une surface interne (2') qui reçoit des inserts et une surface externe (3') dont l'enveloppe géométrique est constituée de plusieurs portions (4') de surfaces ou de sphères de rayon différent, la dernière portion (5') située à l'équateur comportant une ou plusieurs lamelles (6') disposées suivant un arc de cercle, est caractérisé en ce que ces dites lamelles (6') possèdent une surface externe (7') en sur-élévation ou sur-épaisseur par rapport à la surface externe (8') de la portion (5') de l'élément d'ancrage acétabulaire (1').

Selon une autre caractéristique de l'invention, l'implant d'ancrage acétabulaire (1') selon la caractéristique précédente, est caractérisé en ce que la fente (14) pratiquée sous les lamelles (6') détermine des ouvertures (15) sur les côtés (18) et (19) ainsi qu'une ouverture (16) sur le côté (17) de l'élément d'ancrage acétabulaire (1').

Selon une autre caractéristique de l'invention, l'implant d'ancrage acétabulaire (1') suivant les caractéristiques précédentes, est caractérisé en ce que les lamelles (6) ou (6') sont positionnées à des endroits particuliers qui correspondent du côté de l'os, aux cornes du sourcil cotyloïdien.

Les dessins annexés illustrent l'invention :
La figure 1 présente un élément d'ancrage acétabulaire en vue de profil avec un système de lamelles à l'équateur dont les fentes débouchent vers le bas.
La figure 2 est une vue de dessus de la figure 1 de l'élément d'ancrage acétabulaire.
La figure 3 est une coupe suivant AA du même élément d'ancrage.
La figure 4 présente une vue de détail de la coupe AA qui montre le profil d'une lamelle.
La figure 5 présente une vue de face d'une lamelle sur un élément d'ancrage acétabulaire.
La figure 6 montre un autre mode de réalisation des lamelles sur un élément d'ancrage acétabulaire.
La figure 7 est une vue de dessous de la figure 6, montrant en détail une lamelle.
La figure 8 est une section de BB de la figure 7.
La figure 9 est une vue de profil montrant en détail une lamelle.

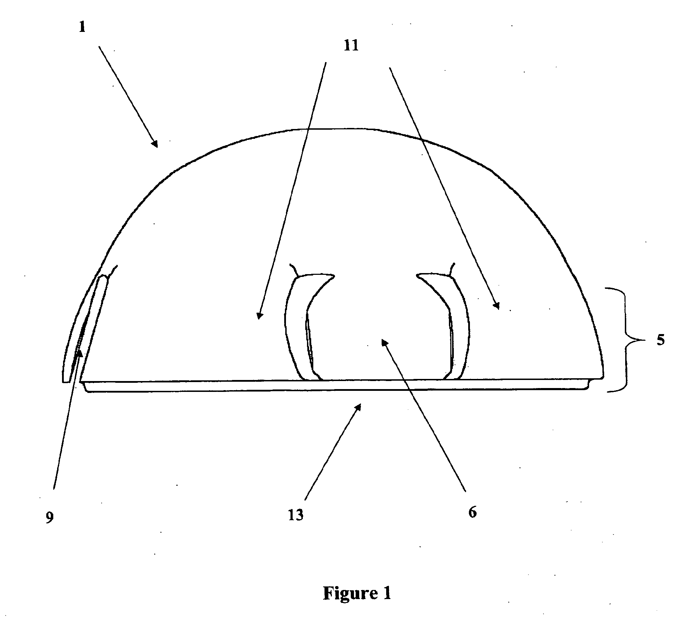

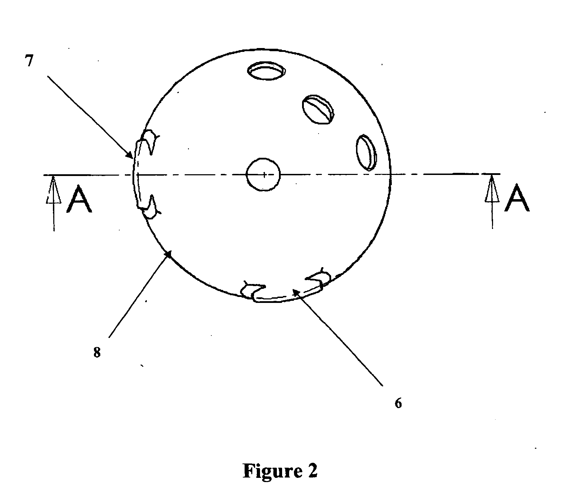

En référence à ces dessins, l'élément d'ancrage (1) présente dans la zone équatoriale externe (5) un système de lamelles (6) présentant chacune une fente (9) débouchant selon la figure 1, vers la partie inférieure de l'élément d'ancrage acétabulaire (13) et latéralement sur les côtés (11). La partie de la zone équatoriale qui est une portion de sphère (5) est représentée dans la figure 2 par la portion (8). La figure 2 montre que la portion de surface (7) de la lamelle (6) est en sur-élévation par rapport à la portion de surface (8).

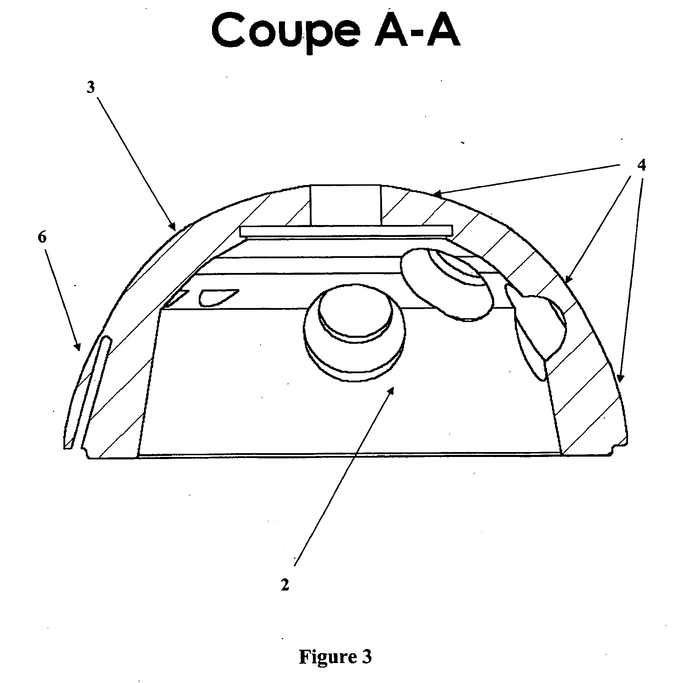

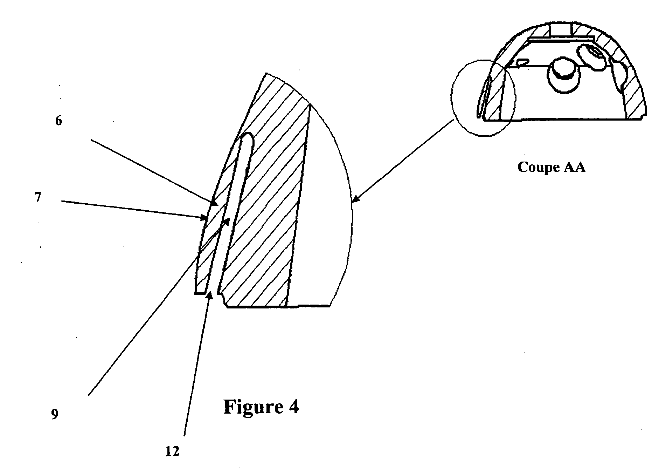

La coupe AA représentée dans la figure 3 et la vue en détail de la figure 4 illustrent ce point. Les portions (4) de surface ont des rayons différents de manière à permettre que la surface (7) de la lamelle (6) soit en sur-élévation ou en sur-épaisseur par rapport à la portion (5) des surfaces (4). Une fente (9) sépare la lamelle (6) du corps de l'élément d'ancrage acétabulaire. Dans cette vue, on aperçoit l'ouverture (12) de cette fente (9).

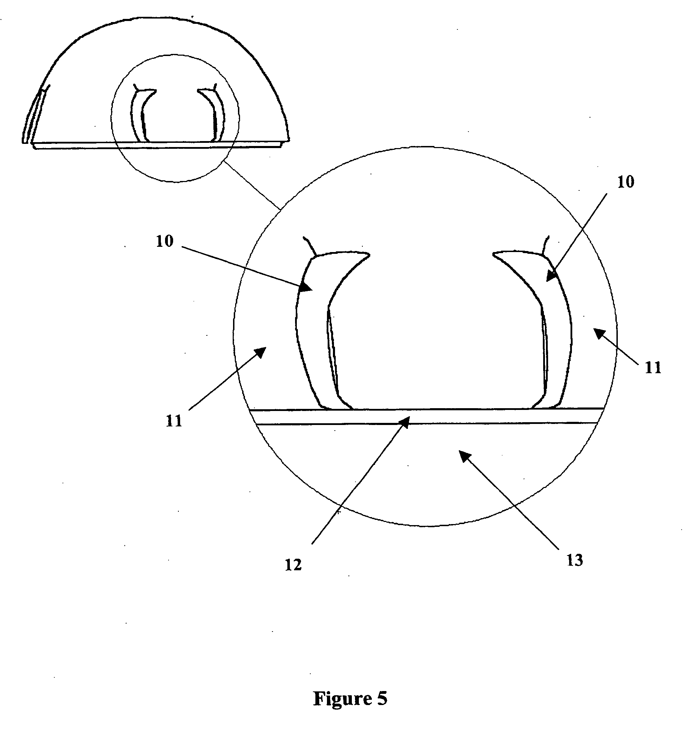

Dans la figure 5, la lamelle est vue de face et présente deux ouvertures sur les côtés (10) et vers le bas (12).

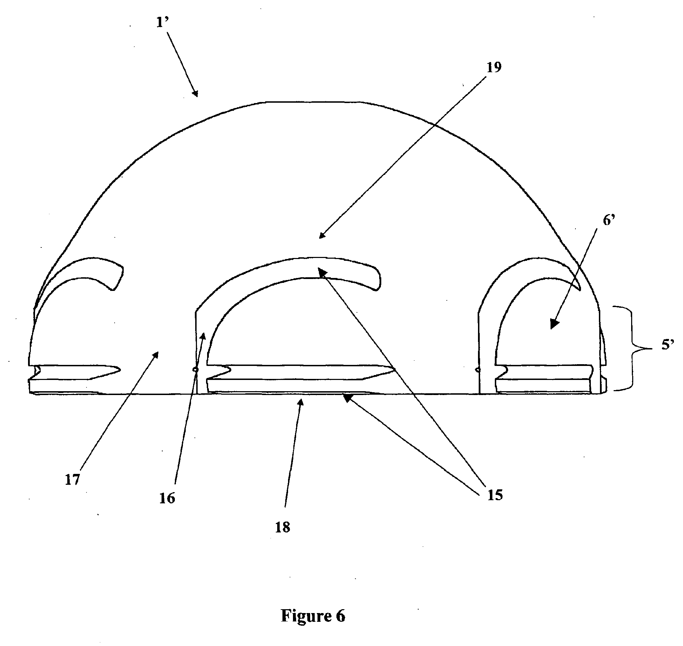

Selon une autre variante, la figure 6 présente un élément d'ancrage acétabulaire (1') comportant un système de lamelles (6') dont les fentes débouchent latéralement sur le côté (17) et sur la face inférieure (18) de l'élément d'ancrage acétabulaire (1') et vers le haut (19).

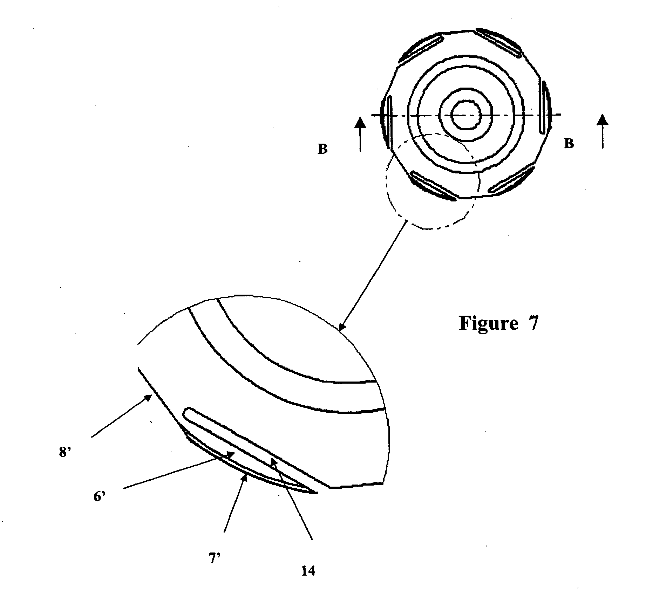

Une partie de la zone équatoriale qui est une portion de surface (5') est représentée dans la figure 7 par la portion (8').

La figure 7 montre que la portion de surface (7') de la lamelle (6') est en sur-élévation par rapport à la portion de surface (8').

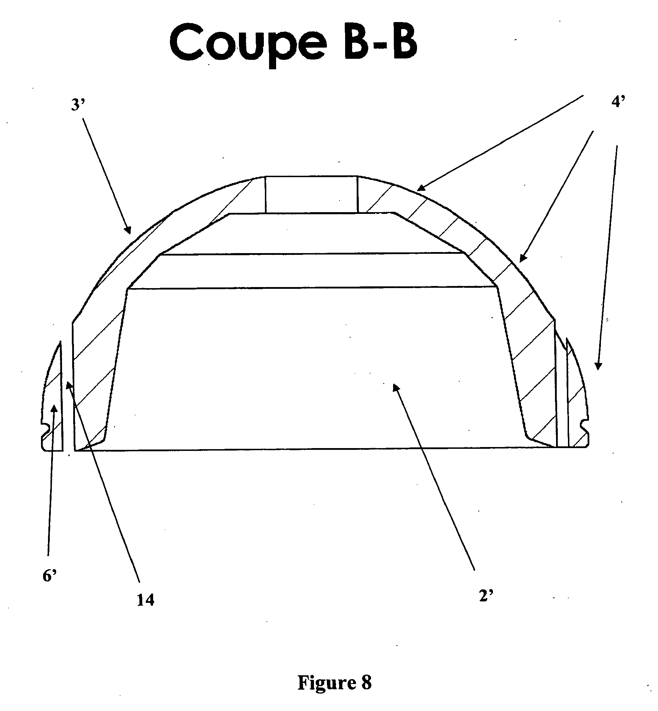

La figure 8 montre en section l'élément d'ancrage acétabulaire (1'). L'élément d'ancrage acétabulaire (1') présente une surface externe (3') et une surface interne (2'). Sa surface externe (3') présente différentes portions de surface (4').

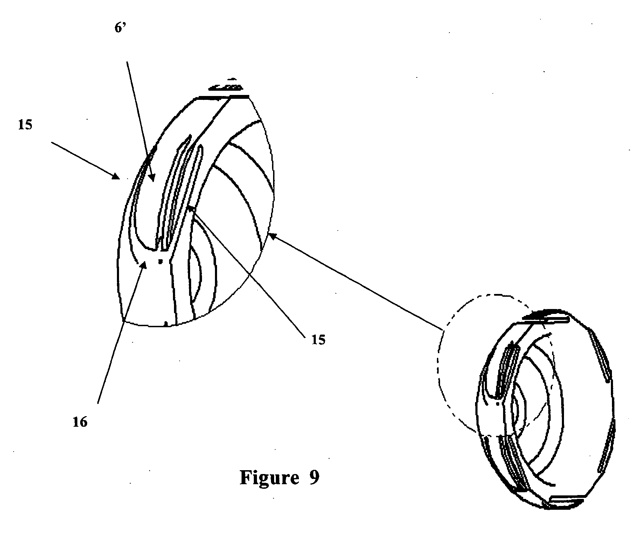

Sur la figure 9, on remarque que la fente (14) débouche sur des ouvertures (15) et (15).

Le nombre des lamelles est supérieur ou égal à 2.

A titre d'exemple non limitatif, les figures 1,2,3,4 et 5 illustrent un mode de réalisation comportant un élément d'ancrage acétabulaire (1) à 2 lamelles dont les fentes débouchent latéralement (11) et vers le bas (13).

Dans une autre variante illustrée par la figure 6,7,8 et 9 l'élément d'ancrage acétabulaire présente un système de 6 lamelles (6') réparties uniformément sur le pourtour équatorial. Ce système permet d'augmenter l'élasticité d'ancrage primaire.

Ces éléments d'ancrage acétabulaire sont particulièrement utilisés dans la chirurgie orthopédique de l'articulation coxo-fémorale.

## Revendications

1. Implant d'ancrage acétabulaire (1,1') destiné à être implanté dans la cavité cotyloïde et à y être ancré par fusion osseuse de forme générale hémisphérique et pouvant recevoir un insert coopérant avec une tête fémorale, ledit élément d'ancrage (1, 1') présentant une surface interne (2, 2') concave, rigide et indéformable recevant l'insert et une surface externe (3, 3') présentant au niveau de sa région équatoriale (5, 5') des moyens de déformation élastique constitués d'une ou plusieurs lamelles (6, 6') disposées suivant un arc de cercle, implant **caractérisé en ce que** la surface externe de la région équatoriale présente en un ou plusieurs endroits une surépaisseur, **en ce que** une ou plusieurs fentes (9, 14) sont pratiquées dans ladite surépaisseur, lesdites fentes débouchant sur la surface externe par des ouvertures (10, 12, 15, 16) afin de former lesdites lamelles (6, 6') et **en ce que** lesdites lamelles (6,6') se présentent en surélévation par rapport à la surface externe de la région équatoriale (5, 5').

2. Implant d'ancrage selon la revendication 1 **caractérisé en ce que** l'épaisseur de la fente (9, 14) et celle de la lamelle (6, 6') sont telles que la déformation initiale liée à l'impaction de la cupule dans la cavité cotyloïdienne n'entame qu'une partie du potentiel de déformation de ladite lamelle.

3. Implant d'ancrage acétabulaire (1) selon la revendication 1 ou 2, **caractérisé en ce qu'**au moins une lamelle (6) est formée par une fente (9) pratiquée au niveau de la surépaisseur de la zone équatoriale et débouchant vers la partie inférieure (13) de l'élément d'ancrage par une ouverture (12) ladite ouverture se prolongeant sur la surface externe par des ouvertures (10) suivant les méridiens de la zone équatoriale de l'implant.

4. Implant d'ancrage acétabulaire (1') selon la revendication 1 ou 2, **caractérisé en ce qu'**au moins une lamelle (6') est formée par une fente (14) pratiquée au niveau de la surépaisseur de la zone équatoriale et débouchant sur la surface externe par une ouverture (16) située suivant un méridien de la zone équatoriale de l'implant reliée à une ouverture (15) globalement parallèle à l'équateur et à une ouverture (15) débouchant vers la partie inférieure (18) de l'implant.

5. Implant d'ancrage acétabulaire (1, 1') selon la,revendication 1 ou 2 **caractérisé en ce qu'**il comporte au moins deux lamelles (6, 6') et **en ce que** les dites lamelles sont situées sur la même moitié du pourtour équatorial.

6. Implant d'ancrage acétabulaire (1, 1') selon la revendication 1 ou 2 **caractérisé en ce qu'**il comporte un système de lamelles (6, 6') réparties uniformément sur le pourtour équatorial.

7. Implant d'ancrage acétabulaire (1, 1') selon la revendication 6 **caractérisé en ce que** le dit système de lamelles comporte six lamelles (6, 6').

## Claims

1. An acetabular anchoring implant (1,1'), designed to be implanted into the pelvle socket and to be anchored therein by a generally hemispherically shaped osseous fusion, and capable of receiving an Insert cooperating with a femoral head, said anchoring member (1, 1') having a concave, rigid, and dimensionally stable internal surface (2, 2') receiving the insert, and an external surface (3, 3') having at the equatorial region (5, 5') thereof means for elastic deformation, composed of one or more lamellae (6, 6') arranged along the arc of a circle, the implant being **characterized in that** the external surface of the equatorial region has a reinforcement in or more positions, **in that** one or more slots (9, 14) are made in said reinforcement, with said slots opening onto the external surface through apertures (10, 12, 15, 16) so as to form said lamellae (6, 6'), and **in that** said lamellae (6, 6') are raised with respect to the external surface of the equatorial region (5, 5').

2. The anchoring implant according to claim 1, **characterized in that** the thickness of the slot (9, 14) and that of the lamella (6, 6') are such that the initial deformation associated with the impaction of the cupula into the pelvic socket only consumes part of the deformation potential of said lamella.

3. The acetabular anchoring implant (1) according to claim 1 or 2, **characterized in that** at least one lamella (6) is formed by a slot (9) made at the reinforcement of the equatorial area, and opening via an aperture (12) onto the lower part (13) of the anchoring member, with said aperture being extended over the external surface by apertures (10) following the meridians of the equatorial area of the implant.

4. The acetabular anchoring implant (1') according to claim 1 or 2, **characterized in that** at least one lamella (6') is formed by a slot (14) made at the reinforcement of the equatorial area and opening onto the external surface via an aperture (16) situated along a meridian of the equatorial area of the implant, which is linked to an aperture (15) globally parallel to the equator, and to an aperture (15) opening onto the lower part (18) of the implant.

5. The acetabular anchoring implant (1, 1') according to claim 1 or 2, **characterized in that** it comprises at least two lamellae (6, 6'), and **in that** said lamellae are located on the same half of the equatorial circumference.

6. The acetabular anchoring implant (1, 1') according to claim 1 or 2, **characterized In that** it comprises an array (6, 6') of lamellae evenly distributed on the equatorial circumference.

7. The acetabular anchoring implant (1, 1') according to claim 6, **characterized in that** the lamella array comprises six lamellae (6, 6').

## Patentansprüche

1. Hüftgelenk-Verankerungsimplantat (1, 1'), das dazu gedacht ist, in die Beckenpfanne implantiert zu werden und darin durch eine allgemein halbkugelförmige Knochenverschmelzung verankert zu werden, und das ein Einsatzstück aufnehmen kann, das mit einem Hüftkopf zusammenwirkt, wobei das Verankerungselement (1, 1') eine konkave, unblegsame und formstabile interne Oberfläche (2, 2'), die das Einsatzstück aufnimmt, und eine externe Oberfläche (3, 3'), die an ihrem äquatorialen Bereich (5, 5') Mittel zur elastischen Verformung aufweist, die aus einer oder mehreren Lamellen (6, 6') bestehen, die an einem Kreisbogen entlang angeordnet sind, aufweist, wobei das Implantat **dadurch gekennzeichnet ist, dass** die externe Oberfläche des äquatorialen Bereichs an einer oder mehreren Stellen eine Verdickung aufweist, dass eine oder mehrere Schlitze (9, 14) in der Verdickung angebracht sind, wobei die Schlitze in die externe Oberfläche durch Öffnungen (10, 12, 15, 16) einmünden, um die Lamellen (6, 6') zu bilden, und dass die Lamellen (6, 6') im Verhältnis zur externen Oberfläche des äquatorialen Bereichs (5, 5') erhöht vorliegen.

2. Verankerungsimplantat nach Anspruch 1, **dadurch gekennzeichnet, dass** die Dicke des Schlitzes (9, 14) und die der Lamelle (6, 6') derart sind, dass die ursprüngliche Verformung, die mit der Impaktion der Schale in der Beckenpfanne verbunden ist, nur einen Tell des Verformungsvermögens der Lamelle verbraucht.

3. Hüftgelenk-Verankerungsimplantat (1) nach Anspruch 1 oder 2, **dadurch gekennzeichnet, dass** mindestens eine Lamelle (6) durch einen Schlitz (9) gebildet wird, der an der Verdickung der äquatorialen Zone angebracht ist, und über eine Öffnung (12) In den unteren Teil (13) des Verankerungselements einmündet, wobei die Öffnung sich auf der externen Oberfläche durch Öffnungen (10) verlängert, die den Meridianen der äquatorialen Zone des Implantats folgen.

4. Hüftgelenk-Verankerungsimplantat (1') nach Anspruch 1 oder 2, **dadurch gekennzeichnet, dass** mindestens eine Lamelle (6') durch einen Schlitz (14) gebildet wird, der an der Verdickung der äquatorialen Zone angebracht wird und in die externe Oberfläche über eine Öffnung (16) einmündet, die sich an einem Meridian der äquatorialen Zone des Implantats entlang befindet, die mit einer Öffnung (15), die insgesamt parallel zum Äquator liegt, und mit einer Öffnung (15), die in den unteren Teil (18) des Implantats einmündet, verbunden ist.

5. Hüftgelenk-Verankerungsimplantat (1, 1') nach Anspruch 1 oder 2, **dadurch gekennzeichnet, dass** es mindestens zwel Lamellen (6, 6') umfasst und dass die beiden Lamellen sich auf derselben Hälfte des äquatorialen Umfangs befinden.

6. Hüftgelenk-Verankerungsimplantat (1, 1') nach Anspruch 1 oder 2, **dadurch gekennzeichnet, dass** es ein System (6, 6') aus Lamellen umfasst, die gleichmäßig auf dem äquatorialen Umfang verteilt sind.

7. Hüftgelenk-Verankerungsimplantat (1, 1') nach Anspruch 6, **dadurch gekennzeichnet, dass** das Lamellensystem sechs Lamellen (6, 6') umfasst.
